Europäisches Patentamt

European Patent Office

Office européen des brevets

(19)

(11) Veröffentlichungsnummer : **0 187 261**
**B1**

# EUROPÄISCHE PATENTSCHRIFT

(12)

(45) Veröffentlichungstag der Patentschrift :
15.03.89

(51) Int. Cl.⁴ : **C 07 D323/06**

(21) Anmeldenummer : **85115334.6**

(22) Anmeldetag : **03.12.85**

(54) Verfahren zur kontinuierlichen Herstellung von Trioxan.

(30) Priorität : **17.12.84 DE 3445921**

(43) Veröffentlichungstag der Anmeldung :
**16.07.86 Patentblatt 86/29**

(45) Bekanntmachung des Hinweises auf die Patenterteilung : **15.03.89 Patentblatt 89/11**

(84) Benannte Vertragsstaaten :
**AT BE CH DE FR GB IT LI NL SE**

(56) Entgegenhaltungen :
EP-A- 0 009 797
EP-A- 0 012 304
EP-A- 0 017 067
DE-B- 2 001 070
FR-A- 1 377 169

(73) Patentinhaber : **HOECHST AKTIENGESELLSCHAFT**
**Postfach 80 03 20**
**D-6230 Frankfurt am Main 80 (DE)**

(72) Erfinder : **Mück, Karl-Friedrich, Dr.**
**Wittenberger Strasse 17**
**D-6200 Wiesbaden (DE)**
Erfinder : **Burg, Karlheinz, Dr.**
**Eichenweg 18**
**D-6200 Wiesbaden (DE)**

EP 0 187 261 B1

**Beschreibung**

Die Herstellung von Trioxan aus wäßrigen Formaldehyd-Lösungen ist verschiedentlich in der Literatur beschrieben (vgl. Walker, Formaldehyde Reinhold Publ. New York, 3. Auflage (1964) Seite 198-199). Hierbei werden wäßrige Formaldehyd-Lösungen bei höheren Temperaturen mit sauren Katalysatoren umgesetzt und das Trioxan durch Destillation aus dem Reaktionsraum entfernt. Als Katalysatoren werden in der Literatur neben Säuren auch Ionenaustauscher empfohlen (vgl. DE-Patentschrift 1 135 491). Der Synthesedampf, der außer Trioxan, Wasser und Formaldehyd noch Nebenprodukte der Synthese enthält, wird dabei fortlaufend aus der Reaktionsmischung entfernt. Dies geschieht durch Rektifikation meist in einer dem Reaktor aufgesetzten Verstärkerkolonne entsprechend der US-Patentschrift 2 304 080 oder in einer Kolonne mit Verstärker- und Abtriebsteil entsprechend der GB-Patentschrift 1 012 372. Die erhaltene trioxanreiche Phase wird dann durch Extraktion und/oder ein anderes bekanntes Trennverfahren weiter aufgearbeitet.

Bei Verwendung von Ionenaustauschern als Katalysatoren wird die Bildung von Nebenprodukten — im Vergleich zum Einsatz von Säuren — zwar reduziert, jedoch sind Ionenaustauscher bei der bisher bekannten Verfahrensweise nur dann störungsfrei einsetzbar, wenn die Konzentration der Formaldehyd-Lösung nicht mehr als ca. 40 Gew.-% beträgt. Bei höheren Formaldehyd-Konzentrationen treten demgegenüber im Reaktor zunehmend Paraformaldehyd-Ablagerungen auf, so daß schon bei ca. 60 GeW.-% Formaldehyd-Konzentration eine kontinuierliche Verfahrensführung über längere Zeiträume nicht mehr möglich ist. Bei niedrigen Formaldehyd-Konzentrationen sind andererseits die erreichbaren Trioxan-Gleichgewichtskonzentrationen so gering, daß ein solches Verfahren nicht wirtschaftlich ist. Weiterhin erweisen sich Ionenaustauscher in herkömmlichen Umlaufreaktoren als mechanisch nicht beständig genug, um lange Reaktorlaufzeiten zu gewährleisten.

Auch gemäß den Verfahren der EP-Patentschriften 0 012 304 und 0 017 067 ist die Menge an Nebenprodukten, insbesondere Ameisensäure und Paraformaldehyd, recht beträchtlich.

Aus der FR-A 1 377 169 ist weiterhin eine Trioxansynthese bekannt, wobei als Emulsionsmittel ein inertes Öl, z. B. Paraffinöl, eingesetzt wird. Durch diese Verfahrensweise soll die Verwertung von hochkonzentriertem Formaldehyd ermöglicht sein. Nachteilig an diesem Verfahren ist jedoch, daß zur Erzeugung der Emulsion eine intensive Mischung mit den entsprechenden Aggregaten erforderlich ist. Der verwendete hohe Säuregehalt führt zudem zu einem höheren Anteil an Nebenprodukten. Außerdem gibt das « inerte » Öl bei Langzeitbetrieb Anlaß zur Bildung prozeßfremder Nebenprodukte und verursacht zusätzliche Probleme bei der Entsorgung des Reaktors.

Die Aufgabe der Erfindung war es daher, ein Verfahren zur kontinuierlichen Herstellung von Trioxan, gegebenenfalls zusammen mit cyclischen Formalen, bereitzustellen, bei dem die beschriebenen Nachteile des Standes der Technik zumindest weitgehend vermieden werden.

Erfindungsgemäß wird dies durch eine Verfahrensweise erreicht, bei der die Umsetzung ohne gleichzeitige Verdampfung erfolgt.

Die vorliegende Erfindung betrifft daher ein Verfahren zur kontinuierlichen Herstellung von Trioxan, gegebenenfalls zusammen mit cyclischen Formalen, durch Trimerisierung von Formaldehyd in wäßriger Lösung, die gegebenenfalls zusätzlich mindestens ein Diol und/oder mindestens ein Epoxid enthält, in Gegenwart von sauren Festbettkatalysatoren, dadurch gekennzeichnet, daß die Umsetzung ohne gleichzeitige Verdampfung durchgeführt wird und anschließend eine Anreicherung des Trixans außerhalb des Reaktors erfolgt.

Nach dem erfindungsgemäßen Verfahren lassen sich überraschender Weise auch hochkonzentrierte Formaldehyd-Lösungen, die bis zu 80 Gew.-% an Formaldehyd enthalten können, zur Reaktion bringen, ohne daß nennenswerte Paraformaldehyd-Ablagerungen auftreten. Die Bildung an Nebenprodukten, wie Ameisensäure, Methylformiat, Methylal, Trioxepan, Tetroxan udgl. ist außerdem sehr gering. Die Trioxangehalte entsprechen dem Gleichgewichtswert. Die erfindungsgemäß eingesetzten Katalysatoren sind außerdem über längere Zeit unter den erfindungsgemäßen Versuchsbedingungen beständig.

Die Trimerisationsreaktion des Formaldehyds zum Trioxan wird erfindungsgemäß ohne gleichzeitige Verdampfung, d. h. ohne Entfernung insbesondere des Trioxans und gegebenenfalls cyclischer Formale aus dem Reaktionssystem, durchgeführt. Die Verweilzeit des Reaktionsgemisches im Reaktor ist erfindungsgemäß recht kurz und liegt — in Abhängigkeit der Reaktionstemperatur — im allgemeinen bei mindestens 0,1 Minuten, zweckmäßigerweise zwischen 0,1 und 15 Minuten, vorzugsweise zwischen 0,9 und 9 Minuten und besonders bevorzugt zwischen 2 und 5 Minuten. Höhere Reaktionstemperaturen haben dabei naturgemäß kürzere Verweilzeiten zur Folge.

Der Formaldehyd-Gehalt in der Zuspeiselösung kann etwa 30 bis etwa 80 Gew.-% betragen ; die besonderen Vorteile zeigt das erfindungsgemäße Verfahren in dem Konzentrationsbereich von etwa 60 bis etwa 80 Gew.-%.

Die Temperaturen, bei denen die Umsetzung erfolgt, liegen je nach Druck zweckmäßigerweise zwischen 80 bis 140 °C, wobei Temperaturen von 90 bis 120 °C bevorzugt sind. Der Druck im Reaktor, der im allgemeinen 0,5 bis 10 bar, vorzugsweise 1 bis 5 bar beträgt, ist gleichfalls wie die Temperatur an und für sich nicht kritisch ; er ist jedoch stets so zu wählen, daß keine Verdampfung im Reaktor stattfindet.

Als Katalysatoren kommen erfindungsgemäß die hierfür bekannten sauren Feststoffe in Betracht, deren Säurestärke entsprechend hoch liegen muß, um eine ausreichende Katalyse der Trimerisierungsreaktion zu gewährleisten. Dies ist beispielsweise bei sulfonsäuregruppenhaltigen Feststoffen der Fall. Derartige für die erfindungsgemäßen Zwecke geeignete sauren Feststoffkatalysatoren sind beispielsweise in der DE-Auslegeschrift 1 135 491, der US-Patentschrift 3 176 023, der GB-Patentschrift 1 012 372, sowie in den DE-Auslegeschriften 1 543 340 und 2 001 070 beschrieben. Beispielhaft seien hier als saure Feststoffkatalysatoren genannt : Sulfonsäuregruppenhaltige Kationenaustauscher, Zeolithe, sogenannte feste « Supersäuren », d. h. Feststoffe auf Basis von wasserhaltigen Metalloxiden wie Eisen (III)-oxid, Zirkondioxid oder Titandioxid, die mit Schwefelsäure imprägniert und anschließend bei höheren Temperaturen calciniert werden. Bevorzugt sind hierbei die sulfonsäuregruppenhaltigen Kationenaustauscher, insbesondere solche auf Basis von vernetztem Polystyrol oder von Fluorpolymeren. Diese Kationenaustauscher können makroporöse Struktur oder Gelstruktur besitzen, wobei letztere bevorzugt ist.

Die Austauscherkapazität dieser Kationenaustauscher liegt im allgemeinen zwischen 0,5 und 5 Val/l gequollener Substanz, vorzugsweise zwischen 0,7 und 2,5 Val/l.

Falls nach der erfindungsgemäßen Variante ein Gemisch aus Trioxan und mindestens einem cyclischen Formal hergestellt werden soll, werden der wäßrigen Formaldehyd-Lösung zweckmäßigerweise 1 bis 25 %, vorzugsweise 2 bis 15 %, bezogen auf Formaldehyd, an mindestens einem Diol und/oder mindestens einem Epoxid zugesetzt. Die hierfür in Frage kommenden Diole sind vor allem 1,2-Diole, 1,3-Diole und α,ω-Diole. Ebenso können statt der 1,2-Diole auch die entsprechenden Epoxide oder Gemische aus beiden eingesetzt werden. Als geeignet haben sich. z. B. Ethylenglykol, Ethylenoxid, Propylenglykol-1,2 ; Propylenoxid, Propylenglykol-1,3, Butandiol-1,2, Butandiol-1,3, Butandiol-1,4 und Buten (3)-diol-1,2 erwiesen. Vorzugsweise werden erfindungsgemäß dabei Ethylenglykol, Ethylenoxid, Propylenglykol-1,2 und Butandiol-1,4 eingesetzt und besonders bevorzugt Ethylenglykol und Ethylenoxid.

Die Reaktion wird erfindungsgemäß in einem Reaktor durchgeführt, der vorzugsweise als Rohrreaktor (Strömungsrohr) ausgebildet ist. Derartige Rohrreaktoren sind in ihrem wesentlichen Aufbau beschrieben in Ullmann, Encyklopädie der technischen Chemie, Verlag Chemie, 4. Auflage 1973, Bd. 3, S. 350-352. Der Reaktor kann dabei auch als Röhrenbündel ausgebildet sein. Zweckmäßigerweise besitzt der Reaktor auch eine Vorheizzone.

Das den Reaktor verlassende Reaktionsgemisch enthält Trioxanmengen, die dem Gleichgewichtswert entsprechen. Der Ameisensäuregehalt, bezogen auf das erzeugte Trioxan, ist zumeist kleiner als 1,5 %. Die Reaktionsmischung wird dann einer Anreicherung des Trioxans und gegebenenfalls der cyclischen Formale unterworfen, was beispielsweise durch Extraktion mit einem geeigneten Extraktionsmittel oder durch Destillation erfolgen kann. Derartige Anreicherungsverfahren sind zum Beispiel in der DE-Auslegeschrift 1 178 082 und der GB-Patentschrift 1 012 372 beschrieben. Auch andere bekannte Anreicherungsverfahren können hierfür Einsatz finden ; verwiesen sei etwa auf Process Program, Stanford Institut Report 23 (1967) S. 181 oder auf die DE-Offenlegungsschrift 1 570 335. Auch die eventuell erforderliche Feinreinigung des Trioxans und der gegebenenfalls gleichzeitig erzeugten cyclischen Formale kann nach bekannten Destillations- und Kristallisations-Methoden erfolgen.

Das erfindungsgemäße Verfahren ist auch verfahrenstechnisch besonders vorteilhaft, da die Reaktion in einfachen Apparaturen durchgeführt und bei kurzen Verweilzeiten gearbeitet werden kann. Die Bildung von Ablagerungen und von Nebenprodukten ist erfindungsgemäß auch bei hohen Formaldehyd-Konzentrationen sehr gering.

Die nachfolgenden Beispiele sollen die Erfindung näher erläutern.

## Beispiele

Die Trimerisierung von Formaldehyd zu Trioxan wurde in einem üblichen Rohreaktor durchgeführt. Er bestand aus einer Vorheizzone und dem eigentlichen Reaktionsraum in Form eines Katalysatorbetts, dessen Volumen 0,167 l betrug. Als Katalysator wurde ein Ionenaustauscher auf Basis von sulfonsäuregruppenhaltigem, vernetztem Polystyrol mit einer Austauscherkapazität von ca. 0,8 Val/l (Handelsprodukt Lewatit° der Fa. BAYER AG) eingesetzt. Die Formaldehyd-Lösung wurde mittels einer Pumpe durch den Reaktor gefördert ; über die Pumpleistung wurde die Verweilzeit im Reaktor gesteuert. Die Reaktionstemperatur im Katalysator wurde mittels eines Thermofühlers registriert und der Systemdruck durch ein Überstromventil eingestellt. Das Reaktionsgemisch in der Vorlage wurde analysiert. Weitere Einzelheiten bezüglich der Reaktionsbedingungen sowie die Ergebnisse sind in der folgenden Tabelle 1 aufgelistet. In dieser sind auch die entsprechendne Daten der Vergleichsversuche enthalten.

Ganz ähnliche Ergebnisse wurden erhalten bei Verwendung eines Katalysators auf Basis von sulfonsäuregruppenhaltigem Polytetrafluoräthylen mit einer Austauscherkapazität von ca. 1,2 Vol/l (Handelsprodukt Nafion° der Fa. DuPONT).

Aus der Tabelle 1 ist ersichtlich, daß im Vergleich zum herkömmlichen Syntheseverfahren in Umlaufreaktoren mit Verdampfern (Vergleichsbeispiele A und C) oder in Reaktoren mit aufgesetzter Kolonne (Vergleichsbeispiel B) im Rohrreaktor mit einem Katalysatorbett ohne Abdestillation des Trioxans (Beispiele 1-8) wesentlich geringere Ameisensäuremengen anfallen und daß auch das Verhältnis von Ameisensäuregehalt und Trioxangehalt wesentlich geringer ist. Das bedeutet, daß die Selektivität der Trioxansynthese wesentlich verbessert wird. Die erhaltenen Trioxangehalte entsprechen den Gleichgewichtswerten der Reaktion, gleichzeitig sind erfindungsgemäß die Verweilzeiten wesentlich geringer als beim herkömmlichen Verfahren.

## Tabelle 1

### Herstellung von Trioxan an Festbettkatalysatoren

| Beispiel | $W_{CH_2O}$ % Dosierung | Temperatur (°C) | Druck (bar) | Verweilzeit (min) | $W_{TOX}$ % Reaktions-Mischung | $W_{HCOOH}$ % Reaktions-Mischung | $\frac{W_{HCOOH} \cdot 100}{W_{TOX}}$ (=Selektivität) | Paraform-aldehyd – Ablagerung |
|---|---|---|---|---|---|---|---|---|
| 1 | 50 | 98 | 1 | 5.5 | 2.2 | 0,02 | 0,9 | – |
| 2 | 65 | 98 | 1 | 5.5 | 4,0 | 0,06 | 1,5 | – |
| 3 | 70 | 98 | 1 | 5.5 . | 5,0 | 0,07 | 1,4 | – |
| 4 | 75 | 98 | 1 | 5.5 | 6,8 | 0,06 | 0,9 | – |
| 5 | 74 | 100 | 3 | 3,6 | 5,9 | 0,08 | 1,4 | – |
| 6 | 74 | 100 | 3 | 3,6 | 5,4 | 0,19 | 3,5 | – |
| 7 | 74 | 100 | 3 | 1,8 | 4,7 | 0,03 | 0,6 | – |
| 8 | 74 | 100 | 3 | 9,0 | 5,9 | 0,15 | 2,6 | – |
| Vergleichsbeispiele | | | | | | | | |
| A[1] | 65 | 100 | 1 | 60 | 3,5 | 0,32 | 9,1 | stark |
| B[2] | 36,5 | 100 | 1 | 180 | | | 7,5*) | nicht be-schrieben |
| C[3] | 60 | 100 | 1 | 40 | | | 5,9 | " |

*) $W_{HCOOH} \times 100 / W_{CH_2O}$ umgesetzt

1) Versuch in einem Umlaufreaktor und Verdampfer gemäß EP-Patentschrift 0 012 304

2) Reaktor mit aufgesetzter Kolonne gemäß DE. Auslegeschrift 1 135 491 (Versuchsdaten aus der Druckschrift entnommen)

3) Beispiel 4 der DE-AS 1 543 340 (Versuchsdatum der Druckschrift entnommen)

**Patentansprüche**

1. Verfahren zur kontinuierlichen Herstellung von Trioxan, gegebenenfalls zusammen mit cyclischen Formalen, durch Trimerisierung von Formaldehyd in wäßriger Lösung, die gegebenenfalls zusätzlich mindestens ein Diol und/oder mindestens ein Epoxid enthält, in Gegenwart von sauren Festbettkatalysatoren, dadurch gekennzeichnet, daß die Umsetzung ohne gleichzeitige Verdampfung durchgeführt wird und anschließend eine Anreicherung des Trioxans außerhalb des Reaktors erfolgt.

2. Verfahren gemäß Anspruch 1, dadurch gekennzeichnet, daß als Katalysator ein Ionenaustauscher auf Basis eines sulfonsäuregruppenhaltigen, vernetzten Polystyrols eingesetzt wird.

3. Verfahren gemäß Anspruch 1, dadurch gekennzeichnet, daß als Festbettkatalysator ein Ionenaustauscher auf Basis eines sulfonsäuregruppenhaltigen Fluorpolymeren eingesetzt wird.

4. Verfahren gemäß mindestens einem der Ansprüche 1 bis 3, dadurch gekennzeichnet, daß die Verweilzeiten zwischen 2 und 5 Minuten liegen.

5. Verfahren nach mindestens einem der Ansprüche 1 bis 4, dadurch gekennzeichnet, daß die Umsetzung in einem Rohrreaktor erfolgt.

6. Verfahren nach mindestens einem der Ansprüche 1 bis 5, dadurch gekennzeichnet, daß die Konzentration der Formaldehydlösung 60 bis 80 Gew.-% beträgt.

**Claims**

1. Process for the continuous preparation of trioxane, optionally together with cyclic formals, by trimerization of formaldehyde in aqueous solution, which optionally contains in addition at least one diol and/or at least one epoxide, in the presence of acidic solid-bed catalysts, wherein the conversion is carried out without simultaneous evaporation and an enrichment of the trioxane is subsequently carried out outside the reactor.

2. A process as claimed in claim 1, wherein an ion exchanger based on a crosslinked polystyrene containing sulfonic acid groups is used as catalyst.

3. A process as claimed in claim 1, wherein an ion exchanger based on a fluoropolymer containing sulfonic acid groups is used as solid-bed catalyst.

4. A process as claimed in at least one of claims 1 to 3, wherein the residence times are between 2 and 5 minutes.

5. A process as claimed in at least one of claims 1 to 4, wherein the conversion takes place in a tubular reactor.

6. A process as claimed in at least one of claims 1 to 5, wherein the concentration of the formaldehyde solution if 60 to 80 % by weight.

**Revendications**

1. Procédé pour préparer en continu du trioxanne, éventuellement avec des formals cycliques, par trimérisation du formaldéhyde en solution aqueuse, qui contient éventuellement en outre au moins un diol et/ou au moins un époxyde, en présence de catalyseurs en lit fixe acide, procédé caractérisé en ce qu'on conduit la réaction sans évaporation simultanée et en ce que l'enrichissement de concentration en trioxanne a lieu ensuite à l'extérieur du réacteur.

2. Procédé selon la revendication 1, caractérisé en ce qu'on utilise comme catalyseur un échangeur d'ions à base d'un polystyrène réticulé contenant des groupes acides sulfoniques.

3. Procédé selon la revendication 1, caractérisé en ce qu'on utilise comme catalyseur en lit fixe un échangeur d'ions à base d'un polymère fluoré contenant des groupes acides sulfoniques.

4. Procédé selon au moins l'une des revendications 1 à 3, caractérisé en ce que les temps de séjour se situent entre 2 et 5 minutes.

5. Procédé selon l'une au moins des revendications 1 à 4, caractérisé en ce que la réaction a lieu dans un réacteur tubulaire.

6. Procédé selon l'une au moins des réactions 1 à 5, caractérisé en ce que la concentration de la solution de formaldéhyde est de 60 à 80 % en poids.